# EUROPEAN PATENT APPLICATION

(11) **EP 4 647 027 A2**
(43) Date of publication of application: **12.11.2025**
(21) Application number: 25204156.1
(22) Date of filing: 02.05.2023
(51) Int. Cl.: A61B 34/00

(54) **CLAMP FORCE CONTROL FEATURE FOR SURGICAL END EFFECTOR**

(30) Priority: 05.05.2022 US 202217737589
(62) Divisional of application: 23726614.3
(71) Applicant: Cilag GmbH International, 6300 Zug (CH)
(72) Inventor: BRUNNER, Eric W., Cincinnati 45242 (US); CLARK, Jeffrey L., Cincinnati 45242 (US); GORDON, Andrew W., Mason 45040 (US); MARRIOTT, Douglas, Mason 45040 (US); MONROE, David A., Cincinnati 45242 (US); TAYLOR, Samantha S., Mason 45040 (US)
(74) Representative: Carpmaels & Ransford LLP

(57) **Abstract**

A surgical instrument and method of compressing tissue includes an end effector and a shaft assembly. The end effector has a jaw and a clamp arm movable between open and closed positions. The shafts assembly includes a clamp actuator having a proximal actuator portion, a distal actuator portion, and a force limiter. The force limiter is longitudinally connected between the proximal and distal actuator portions and configured to transfer movement from the proximal actuator portion to the distal actuator portion up to transferring a predetermined force therethrough. At least a portion of the force limiter is configured to deflect upon exceeding the predetermined force such that the proximal actuator portion is configured to longitudinally move relative to the distal actuator portion and thereby limit a clamping force between the jaw and the clamp arm.

## Description

### BACKGROUND

A variety of surgical instruments include an end effector for use in conventional medical treatments and procedures conducted by a medical professional operator, as well as applications in robotically assisted surgeries. Such surgical instruments may be directly gripped and manipulated by a surgeon or incorporated into robotically assisted surgery. In the case of robotically assisted surgery, the surgeon may operate a master controller to remotely control the motion of such surgical instruments at a surgical site. The controller may be separated from the patient by a significant distance (e.g., across the operating room, in a different room, or in a completely different building than the patient). Alternatively, a controller may be positioned quite near the patient in the operating room. Regardless, the controller may include one or more hand input devices (such as joysticks, exoskeletal gloves, master manipulators, or the like), which are coupled by a servo mechanism to the surgical instrument. In one example, a servo motor moves a manipulator supporting the surgical instrument based on the surgeon's manipulation of the hand input devices. During the surgery, the surgeon may employ, via a robotic surgical system, a variety of surgical instruments including an ultrasonic blade, a tissue grasper, a needle driver, an electrosurgical cautery probes, etc. Each of these structures performs functions for the surgeon, for example, cutting tissue, coagulating tissue, holding or driving a needle, grasping a blood vessel, dissecting tissue, or cauterizing tissue.

In one example, the end effector of the surgical instrument includes a blade element that vibrates at ultrasonic frequencies to cut and/or seal tissue (e.g., by denaturing proteins in tissue cells). These instruments include one or more piezoelectric elements that convert electrical power into ultrasonic vibrations, which are communicated along an acoustic waveguide to the blade element. The precision of cutting and coagulation may be controlled by the operator's technique and adjusting the power level, blade edge angle, tissue traction, and blade pressure. The power level used to drive the blade element may be varied (e.g., in real time) based on sensed parameters such as tissue impedance, tissue temperature, tissue thickness, and/or other factors. Some instruments have a clamp arm and clamp pad for grasping tissue with the blade element. Examples of ultrasonic surgical instruments and related concepts are disclosed in U.S. Pub. No. 2006/0079874, entitled "Tissue Pad for Use with an Ultrasonic Surgical Instrument," published April 13, 2006, now abandoned, the disclosure of which is incorporated by reference herein; U.S. Pub. No. 2007/0191713, entitled "Ultrasonic Device for Cutting and Coagulating," published August 16, 2007, now abandoned, the disclosure of which is incorporated by reference herein; and U.S. Pub. No. 2008/0200940, entitled "Ultrasonic Device for Cutting and Coagulating," published August 21, 2008, now abandoned, the disclosure of which is incorporated by reference herein.

Examples of robotic systems, at least some of which have ultrasonic features and/or associated articulatable portions, include U.S. Pat. App. No. 16/556,661, entitled "Ultrasonic Surgical Instrument with a Multi-Planar Articulating Shaft Assembly," filed on August 30, 2019; U.S. Pat. App. No. 16/556,667, entitled "Ultrasonic Transducer Alignment of an Articulating Ultrasonic Surgical Instrument," filed on August 30, 2019; U.S. Pat. App. No. 16/556,625, entitled "Ultrasonic Surgical Instrument with Axisymmetric Clamping," filed on August 30, 2019; U.S. Pat. App. No. 16/556,635, entitled "Ultrasonic Blade and Clamp Arm Alignment Features," filed on August 30, 2019; U.S. Pat. App. No. 16/556,727, entitled "Rotatable Linear Actuation Mechanism," filed on August 30, 2019; and/or U.S. Pat. App. No. 62/930,638, entitled "Articulation Joint with Helical Lumen," filed on November 5, 2019. The disclosure of each of these applications is incorporated by reference herein.

Some instruments are operable to seal tissue by applying radiofrequency (RF) electrosurgical energy to the tissue. Examples of such devices and related concepts are disclosed in U.S. Pat. No. 7,354,440, entitled "Electrosurgical Instrument and Method of Use," issued April 8, 2008, the disclosure of which is incorporated by reference herein; U.S. Pat. No. 7,381,209, entitled "Electrosurgical Instrument," issued June 3, 2008, the disclosure of which is incorporated by reference herein.

Some instruments are capable of applying both ultrasonic energy and RF electrosurgical energy to tissue. Examples of such instruments are described in U.S. Pat. No. 9,949,785, entitled "Ultrasonic Surgical Instrument with Electrosurgical Feature," issued April 24, 2018, the disclosure of which is incorporated by reference herein; and U.S. Pat. No. 8,663,220, entitled "Ultrasonic Surgical Instruments," issued March 4, 2014, the disclosure of which is incorporated by reference herein.

While several surgical instruments and systems have been made and used, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a front perspective view of a first example of an ultrasonic surgical instrument having a first end effector, a shaft assembly having a first distal shaft portion, and a base assembly configured to connect to a robotic driven interface;
FIG. 2 depicts a rear perspective view of the ultrasonic surgical instrument of FIG. 1;
FIG. 3A depicts an enlarged perspective view of the ultrasonic surgical instrument of FIG. 1 with the end effector in a closed position and the shaft assembly in a straight configuration;
FIG. 3B depicts the enlarged perspective view of the ultrasonic surgical instrument similar to FIG. 3A, but showing the end effector in an open position;
FIG. 4A depicts an enlarged perspective view of the ultrasonic surgical instrument of FIG. 1 with the end effector in a closed position and the shaft assembly in a first articulated configuration;
FIG. 4B depicts the enlarged perspective view of the ultrasonic surgical instrument similar to FIG. 4A, but with the shaft assembly in a second articulated configuration;
FIG. 5 depicts a front perspective view of the ultrasonic surgical instrument of FIG. 1 including a clamp actuator having an exemplary force limiter for limiting clamp closure force with various feature hidden for greater clarity;
FIG. 6 depicts an enlarged front perspective view of the force limiter of FIG. 5;
FIG. 7 depicts an enlarged, partially exploded front perspective view of the force limiter of FIG. 5 including a resilient portion, a spacer, and distal and proximal securements for connecting the spacer relative to the resilient portion;
FIG. 8 depicts an enlarged, exploded front perspective view of the spacer of FIG. 7;
FIG. 9 depicts an enlarged, partially exploded front perspective view of the resilient portion of FIG. 7;
FIG. 10 depicts an enlarged, side elevational view of the resilient portion of FIG. 7;
FIG. 11 depicts a cross-sectional view of the force limiter of FIG. 6 taken along section line 11-11 of FIG. 6 showing an acoustic waveguide within clamp actuator with the clamp arm in the open position of FIG. 3B;
FIG. 12A depicts the cross-sectional view of the force limiter similar to FIG. 11, but with the clamp actuator being pulled in a proximal direction with forces transferred through the resilient portion less than or equal to a predetermined force to, in turn, move the clamp arm from the open position of FIG. 3B toward the closed position of FIG. 3A;
FIG. 12B depicts the cross-sectional view of the force limiter similar to FIG. 12A, but with the clamp actuator being pulled in a proximal direction with forces exceeding the predetermined force while closing the clamp arm against a tissue for limiting clamp closure force; and
FIG. 13 depicts the cross-sectional view of the force limiter similar to FIG. 11, but with the clamp actuator being pushed in a distal direction with forces transferred through the spacer to, in turn, move the clamp arm from the closed position of FIG. 3A toward the open position of FIG. 3B.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "front," "rear," "clockwise," "counterclockwise," "longitudinal," "transverse," "upper," and "lower" also are used herein for reference to relative positions and directions. Such terms are used below with reference to views as illustrated for clarity and are not intended to limit the invention described herein.

### I. Exemplary Surgical Instrument

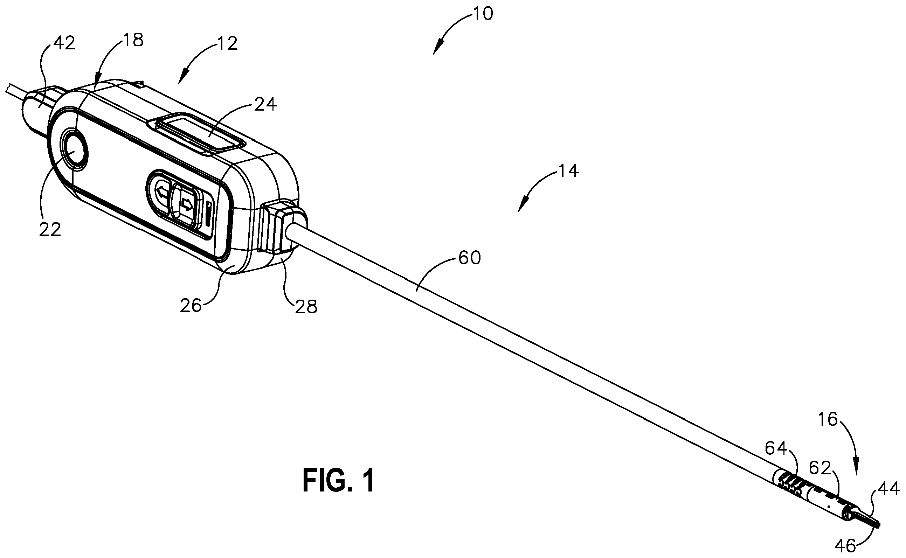

FIG. 1 shows an exemplary surgical instrument, such as a first example of an ultrasonic surgical instrument (10). At least part of ultrasonic surgical instrument (10) may be constructed and operable in accordance with at least some of the teachings of any of the various patents, patent application publications, and patent applications that are cited herein. As described therein and as will be described in greater detail below, ultrasonic surgical instrument (10) is operable to cut tissue and seal or weld tissue (e.g., a blood vessel, etc.) substantially simultaneously. While the present example incorporates various ultrasonic features as ultrasonic surgical instrument (10), the invention is not intended to be unnecessarily limited to the ultrasonic features described herein.

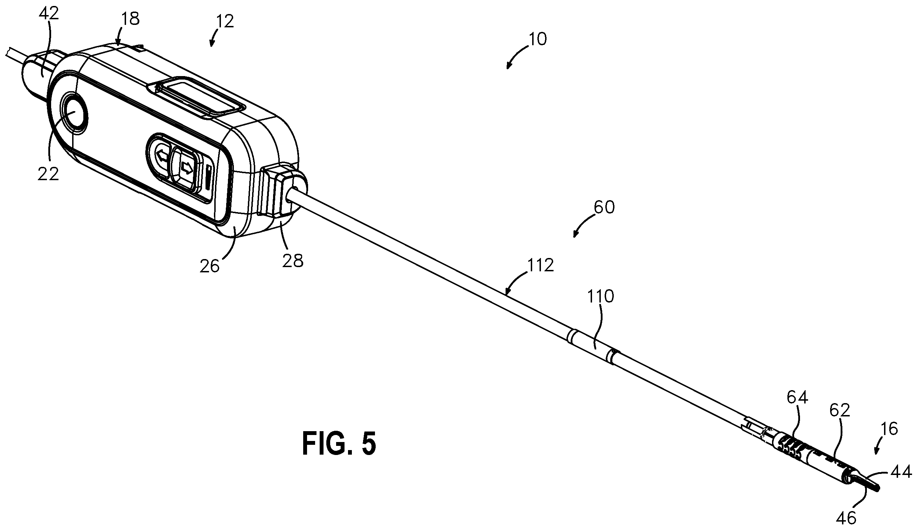

Ultrasonic surgical instrument (10) of the present example comprises a body assembly, such as a base assembly (12), a shaft assembly (14), and an end effector (16). Base assembly (12) includes a housing (18), a button (22), and a pair of latch clasps (24). Button (22) is operatively connected to an electrical base power controller (not shown) and configured to selectively power ultrasonic surgical instrument (10) for use. In addition, housing (18) of the present example includes a front housing cover (26) and a rear housing cover (28) removably secured together via latch clasps (24). More particularly, latch clasps (24) removably secure front housing cover (26) to rear housing cover (28) such that front housing cover (26) may be removed for accessing an interior space (30) (*see* FIG. 5) within base assembly (12). Shaft assembly (14) distally extends from base assembly (12) to end effector (16) to thereby communicate mechanical and/or electrical forces therebetween for use as will be discussed below in greater detail. As shown in the present example, base assembly (12) is configured to operatively connect to a robotic drive (not shown) for driving various features of shaft assembly (14) and/or end effector (16). However, in another example, body assembly may alternatively include a handle assembly (not shown), which may include a pistol grip (not shown) in one example, configured to be directly gripped and manipulated by the surgeon for driving various features of shaft assembly (14) and/or end effector (16). The invention is thus not intended to be unnecessarily limited to use with base assembly (12) and the robotic drive (not shown).

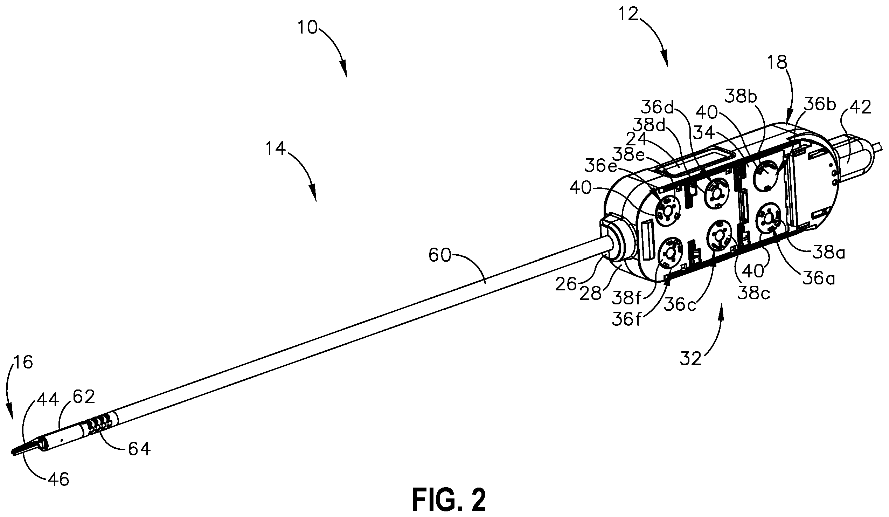

To this end, with respect to FIG. 2, base assembly (12) includes a robotic driven interface (32) extending through a base plate (34) of rear housing cover (28) and configured to mechanically couple with the robotic drive (not shown). Robotic driven interface (32) of the present example includes a plurality of instrument actuators (36a, 36b, 36c, 36d, 36e, 36f) having a plurality of input bodies (38a, 38b, 38c, 38d, 38e, 38f), respectively. Each input body (38a, 38b, 38c, 38d, 38e, 38f), which may also be referred to herein as a "puck," is configured to removably connect with the robotic drive (not shown) and, in the present example, is generally cylindrical and rotatable about an axis. Input bodies (38a, 38b, 38c, 38d, 38e, 38f) have a plurality of slots (40) configured to receive portions of the robotic drive (not shown) for gripping and rotatably driving input bodies (38a, 38b, 38c, 38d, 38e, 38f) in order to direct operation of shaft assembly (14) and/or end effector (16) as will be discussed below in greater detail. Base assembly (12) also receives an electrical plug (42) operatively connected to an electrical power source (not shown) to provide electrical power to base assembly (12) for operation as desired, such as powering electrical base power controller (not shown) and directing electrical energy to various features of shaft assembly (14) or end effector (16) associated with cutting, sealing, or welding tissue.

### A. Exemplary End Effector and Acoustic Drivetrain

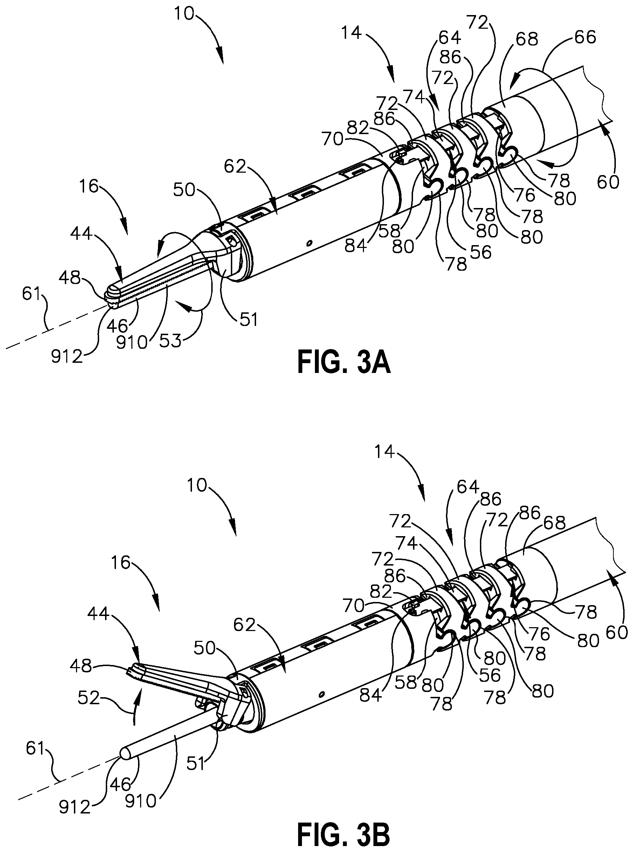

As best seen in FIGS. 3A and 3B, end effector (16) of the present example includes a clamp arm (44) and an ultrasonic blade (46). Clamp arm (44) has a clamp pad (48) secured to an underside of clamp arm (44), facing blade (46). In one example, clamp pad (48) may comprise polytetrafluoroethylene (PTFE) and/or any other suitable material(s). Clamp arm (44) is pivotally secured to a distally projecting tongue (50) of shaft assembly (14). Clamp arm (44) is operable to selectively pivot toward and away from blade (46) to selectively clamp tissue between clamp arm (44) and blade (46). A pair of arms (51) extend transversely from clamp arm (44) and are pivotally secured to another portion of shaft assembly (14) configured to longitudinally slide to pivot clamp arm (44) as indicated by an arrow (52) between a closed position shown in FIG. 3A and an open position shown in FIG. 3B.

In addition to pivoting relative to blade (46), clamp arm (44) of the present example is further configured to rotate about blade (46) relative to blade (46) and also relative to shaft assembly (14) as indicated by an arrow (53). In one example, clamp arm (44) rotates in the clockwise or counterclockwise directions completely around blade (46) and may be selectively fixed in any angular position relative to blade (46) for directing clamp arm (44) from the open position to the closed position for clamping tissue. In another example, clamp arm (44) may have rotational stops (not shown) configured to limit rotational movement of clamp arm (44) relative to blade (46) in one or more predetermined positions.

Blade (46) of the present example is operable to vibrate at ultrasonic frequencies in order to effectively cut through and seal tissue, particularly when the tissue is being compressed between clamp pad (48) and blade (46). Blade (46) is positioned at a distal end of an acoustic drivetrain. This acoustic drivetrain includes a transducer assembly (not shown) and an acoustic waveguide (56), which includes a flexible portion (58) discussed below in greater detail. It should be understood that waveguide (56) may be configured to amplify mechanical vibrations transmitted through waveguide (56). Furthermore, waveguide (56) may include features operable to control the gain of the longitudinal vibrations along waveguide (56) and/or features to tune waveguide (56) to the resonant frequency of the system. Various suitable ways in which waveguide (56) may be mechanically and acoustically coupled with transducer assembly (not shown) will be apparent to those of ordinary skill in the art in view of the teachings herein.

Those of ordinary skill in the art will understand that, as a matter of physics, a distal end of blade (46) is located at a position corresponding to an anti-node associated with resonant ultrasonic vibrations communicated through flexible portion (58) of waveguide (56). When transducer assembly (not shown) is energized, the distal end of blade (46) is configured to move longitudinally in the range of, for example, approximately 10 to 500 microns peak-to-peak, and in some instances in the range of about 20 to about 200 microns at a predetermined vibratory frequency fₒ of, for example, 55.5 kHz. When transducer assembly (not shown) of the present example is activated, these mechanical oscillations are transmitted through waveguide (56) to reach blade (46), thereby providing oscillation of blade (46) at the resonant ultrasonic frequency. Thus, when tissue is secured between blade (46) and clamp pad (48), the ultrasonic oscillation of blade (46) may simultaneously sever the tissue and denature the proteins in adjacent tissue cells, thereby providing a coagulative effect with relatively little thermal spread. In some versions, end effector (16) is operable to apply radiofrequency (RF) electrosurgical energy to tissue in addition to applying ultrasonic energy to tissue. In any case, other suitable configurations for an acoustic transmission assembly and transducer assembly (54) will be apparent to one of ordinary skill in the art in view of the teachings herein. Similarly, other suitable configurations for end effector (16) will be apparent to those of ordinary skill in the art in view of the teachings herein.

### B. Exemplary Shaft Assembly and Articulation Section

As shown in FIGS. 3A and 3B, shaft assembly (14) includes a proximal shaft portion (60) extending along a longitudinal axis (61), a first distal shaft portion (62) distally projecting relative to the proximal shaft portion (60), and an articulation section (64) extending between proximal and distal shaft portions (60, 62). Shaft assembly (14) is configured to rotate about longitudinal axis (61) as indicated by an arrow (66). In one example, shaft assembly (14) rotates in the clockwise or counterclockwise directions completely around longitudinal axis (61) and may be selectively fixed in any rotational position about longitudinal axis (61) for positioning articulation section (64) and/or end effector (16) about longitudinal axis (61). While end effector (16) generally rotates with shaft assembly (14) as indicated by arrow (66), end effector (16) may be simultaneously and independently rotated as indicated by arrow (53) relative to shaft assembly (14) during use for repositioning portions of shaft assembly (14) and/or end effector (16) as desired.

Articulation section (64) is configured to selectively position end effector (16) at various lateral deflection angles relative to longitudinal axis (61) defined by proximal shaft portion (60). Articulation section (64) may take a variety of forms. In the present example, articulation section (64) includes a proximal link (68), a distal link (70), and a plurality of intermediate links (72) connected in series between proximal and distal links (68, 70). Articulation section (64) further includes a pair of articulation bands (74) extending along a pair of respective channels (76) collectively defined through links (68, 70, 72). Links (68, 70, 72) are generally configured to pivot relative to each other upon actuation of articulation bands (74) to thereby bend articulation section (64) with flexible portion (58) of waveguide (56) therein to achieve an articulated state. By way of example only, articulation section (64) may alternatively or additionally be configured in accordance with one or more teachings of U.S. Pat. No. 9,402,682, entitled "Articulation Joint Features for Articulating Surgical Device," issued August 2, 2016, the disclosure of which is incorporated by reference herein. As another merely illustrative example, articulation section (64) may alternatively or additionally be configured in accordance with one or more teachings of U.S. Pat. No. 9,393,037, issued July 19, 2016, entitled "Surgical Instruments with Articulating Shafts," the disclosure of which is incorporated by reference herein and U.S. Pat. No. 9,095,367, issued August 4, 2015, entitled "Flexible Harmonic Waveguides/Blades for Surgical Instruments," the disclosure of which is incorporated by reference herein. In addition to or in lieu of the foregoing, articulation section (64) and/or may be constructed and/or operable in accordance with at least some of the teachings of U.S. Pat. No. 10,034,683, entitled "Ultrasonic Surgical Instrument with Rigidizing Articulation Drive Members," issued on July 31, 2018. Alternatively, articulation section (64) may be constructed and/or operable in any other suitable fashion.

Links (68, 70, 72) shown in FIGS. 3B-4B pivotally interlock to secure distal shaft portion (62) relative to proximal shaft portion (60) while allowing for deflection of distal shaft portion (62) relative to longitudinal axis (61). In the present example, proximal link (68) is rigidly connected to proximal shaft portion (60) and has a pair of arcuate grooves (78) opposed from each other. Intermediate links (72) respectively have a pair of arcuate tongues (80) proximally extending therefrom and a pair of arcuate grooves (78) positioned distally opposite from respective tongues (80). Each intermediate link (72) has tongues (80) pivotally received within adjacent arcuate grooves (78) of another intermediate link (72) or proximal link (68) as applicable. Distal link (70) is rigidly connected to distal shaft portion (62) and has another pair of arcuate tongues (80) opposed from each other and pivotally received within adjacent arcuate grooves (78) of intermediate link (72). Tongues (80) and grooves (78) connect together to form the series of interlocked links (68, 70, 72).

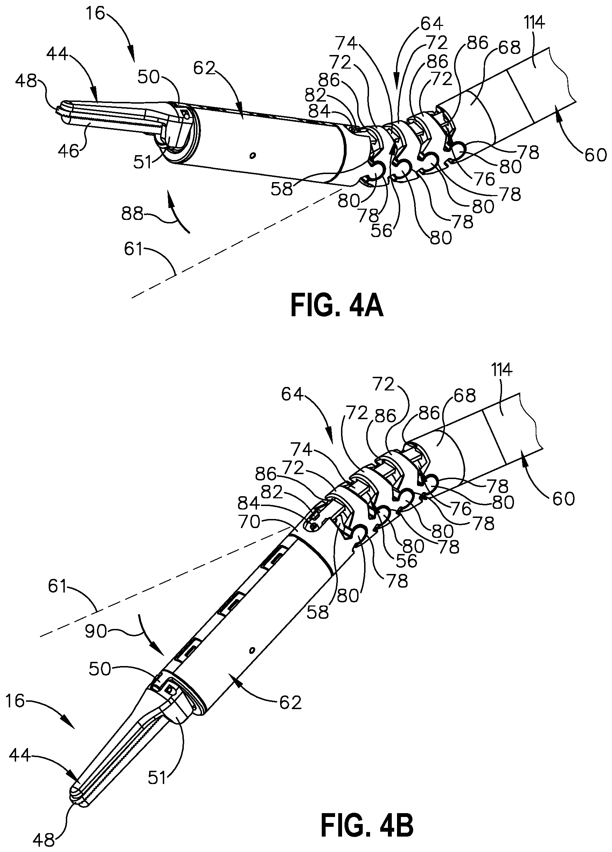

Distal link (70) further includes a pair of opposing notches (82) with a pin (84) therein configured to receive distal end portions of respective articulation bands (74). More particularly, pins (84) extend through a hole in each respective articulation bands (74) while distal end portions of respective articulation bands (74) are coupled within notches (82). Slots (86) in each of intermediate and proximal links (72, 68) longitudinally align with each other and notches (82) to collectively define channels (76) configured to receive articulation bands (74) while allowing articulation bands (74) to slide relative to links (68, 70, 72). To this end, when articulation bands (74) translate longitudinally in an opposing fashion, this will cause articulation section (64) to bend, thereby laterally deflecting end effector (16) away from the longitudinal axis (61) of proximal shaft portion (60) from a straight configuration as shown in FIG. 3B to a first articulated configuration as shown in FIG. 4A and indicated by an arrow (88) or a second articulated configuration as shown in FIG. 4B and indicated by an arrow (90). In particular, end effector (16) will be articulated toward the articulation band (74) that is being pulled proximally. During such articulation, the other articulation band (74) may be pulled distally. Alternatively, the other articulation band (74) may be driven distally by an articulation control. Furthermore, flexible acoustic waveguide (56) is configured to effectively communicate ultrasonic vibrations from waveguide (56) to blade (46) even when articulation section (64) is in an articulated configuration as shown in FIGS. 4A- and 4B.

### II. Exemplary Shaft Assembly with Force Limiter for Limiting Clamp Compression Force

With respect to FIG. 5, while shaft assembly (14) is configured to articulate relative to longitudinal axis (61) for enhanced positioning of end effector (16), proximal shaft portion (60) of the present example also includes an exemplary force limiter (110) operatively connected to clamp arm (44) to limit clamping force to a predetermined maximum clamping force between blade (46) and clamp arm (44) while closing clamp arm toward blade (46). For example, applications of energy, such as ultrasonic energy via blade (46), to tissue may be pressure sensitive with outcomes varying greatly if too much compressive force is applied to tissue during the application of such energy. It is thus desirable in some instances to limit such compressive force to a predetermined maximum clamping force, which may be set and/or adjusted as preferred by an operator of ultrasonic surgical instrument (10). By way of further example, it may be further desired to use the same end effector (16) to pry apart various tissues by opening clamp arm (44) from the closed position toward the open position without such force limitations. In this respect, shaft assembly (14) of the present example is configured to bypass portions of force limiter (110) to allow for differing force applications between compression and prying of tissues as discussed below in greater detail.

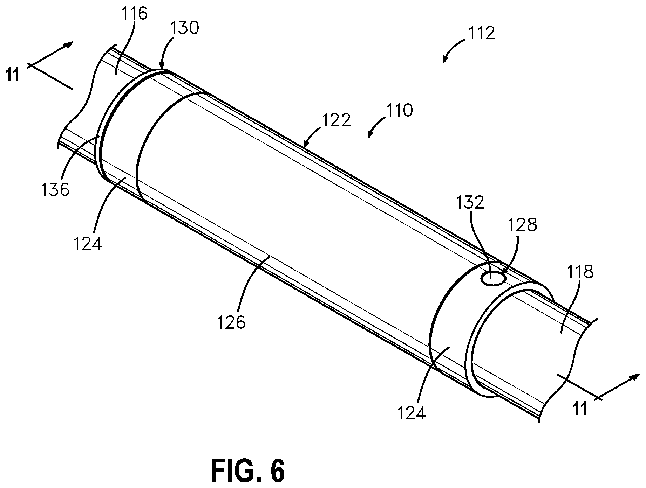

FIGS. 5 and 6 show a clamp actuator (112) in the form of an inner tube of shaft assembly (14) operatively connected to clamp arm (44) for selective movement of clamp arm (44). Outer sheath (114) (*see* FIGS. 4A and 4B) of proximal shaft portion (60) has been removed for clarity. In the present example, selective pulling of clamp actuator (112) in the proximal direction is configured to move clamp arm (44) from the open position toward the closed position, whereas selective pushing of clamp actuator (112) in the distal direction is configured to move clamp arm (44) from the closed position toward the open position. Clamp actuator (112) more particularly includes a proximal actuator portion (116), a distal actuator portion (118), and force limiter (110) longitudinally connected therebetween. In this respect, while closing clamp arm (44), force limiter (110) is configured to transfer movement from the proximal articulator portion (116) to the distal articulator portion (118) up to transferring a predetermined force therethrough. Upon exceeding such predetermined force while closing clamp arm (44), at least a portion of force limiter (110) is configured to deflect such that proximal actuator portion (116) is configured to longitudinally move relative to distal actuator portion (118) and thereby limit the clamping force between blade (46) and clamp arm (44) to the predetermined maximum clamping force.

As shown in the present example, proximal actuator portion (116) extends from base assembly (12) with robotic driven interface (32) for selectively directing proximal and distal movement of clamp actuator (112). Alternatively, proximal actuator portion (16) may extend from a handle assembly (not shown) of a handheld surgical instrument (not shown). In this respect, proximal actuator portion (16) may extend from any body for robotic and/or handheld operation, and the invention is not intended to be unnecessarily limited to robotic applications. This body, and more particularly base assembly (12) in the present example, does not include a resiliently biased element, such as a wave spring, contained therein to limit clamp force closure. Rather, force limiter (110) includes a resilient portion associated with shaft assembly (14) and positioned longitudinally between base assembly (12) and end effector (16). In the present example, force limiter (110) is more particularly shown proximally positioned relative to articulation section (64). In another example, force limiter (110) may be positioned in articulation section (64). In still another example, force limiter (110) may be distally positioned relative to articulation section (64).

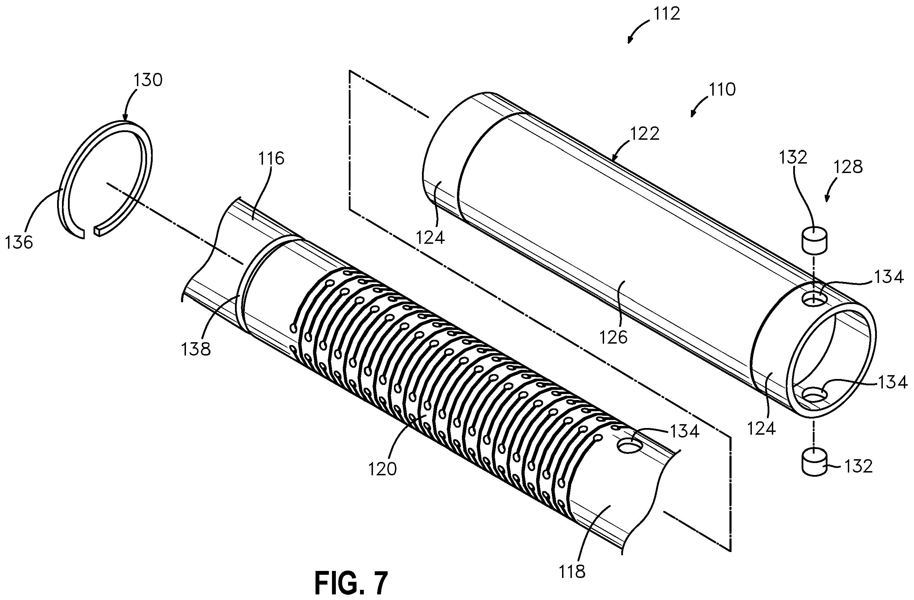

FIGS. 6 and 7 show force limiter (110) of the present example in greater detail including resilient portion, which is shown more particularly as a spring (120), radially surrounded by a spacer (122). While spring (120) generally provides resilient bias for limiting clamp closure force, spacer (122) is configured to set a preloaded tension, such as a 20 lb preload, in spring (120) as discussed below in greater detail. One or more collars (124) of spacer (122) may be added to distal or proximal ends of a spacer body (126) as desired to further elongate a length of the overall spacer (122) for additional preloaded tension in spring (120). Collars (124) are attached to spacer body (126) in the present example, although may be detached in another example. In still another example, at least one of collars (124) may threadably connect to spacer body (126) to shorten or elongate the length of the overall spacer (122) for greater adjustability of preset loading of spring (120).

To this end, force limiter (110) further includes a distal securement (128) and a proximal securement (130) to retain spacer (122) in compression about spring (120) such that spacer (122), in turn, holds spring (120) in tension. In the present example, distal securement (128) includes a pair of upper and lower pins (132) extending through upper and lower holes (134) in spacer (122) and distal actuator portion (118). Pins (132) thus affix spacer (122) relative to distal actuator portion (118) such that no distal or proximal movement occurs between spacer (122) and distal actuator portion (118) during translation of clamp actuator (112) while moving clamp arm (44) regardless of force applied through force limiter (110) during use. In contrast, proximal securement (130) of the present example includes a retaining ring (136) received in an annular channel (138) about proximal actuator portion (116). Retaining ring (126) thereby provides a proximal stop to compress spacer (122) between retaining ring (126) and pins (132) when spacer (122) is seated against retaining ring (126).

Unlike pins (132), retaining ring (126) allows spacer (122) to unseat from retaining ring (126) when spring (120) extends in response to applications of force during use that exceed the predetermined force for limiting clamp compression. While the present example shows distal securement (128) affixing spacer (122) to distal actuator portion (118) and proximal securement (128) allowing some movement of spacer (122) relative to proximal actuator portion (116), it will be appreciated that such securements (128, 138) may be reversed such that proximal securement (130) affixes spacer (122) to proximal actuator portion (116) and distal securement (128) allows some movement of spacer (122) relative to distal actuator portion (118). Also, alternative securements, such as welds, threaded members, and turnbuckles, may be similarly used to provide for spacing of spring (120) and related adjustment of such spacing as discussed above. The invention is thus not intended to be unnecessarily limited to the particular securements of the present example.

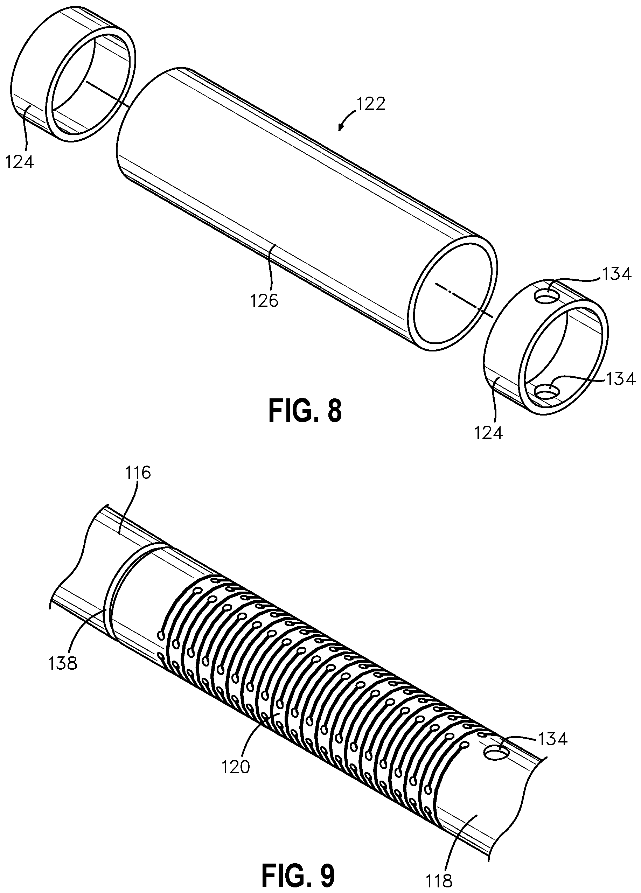

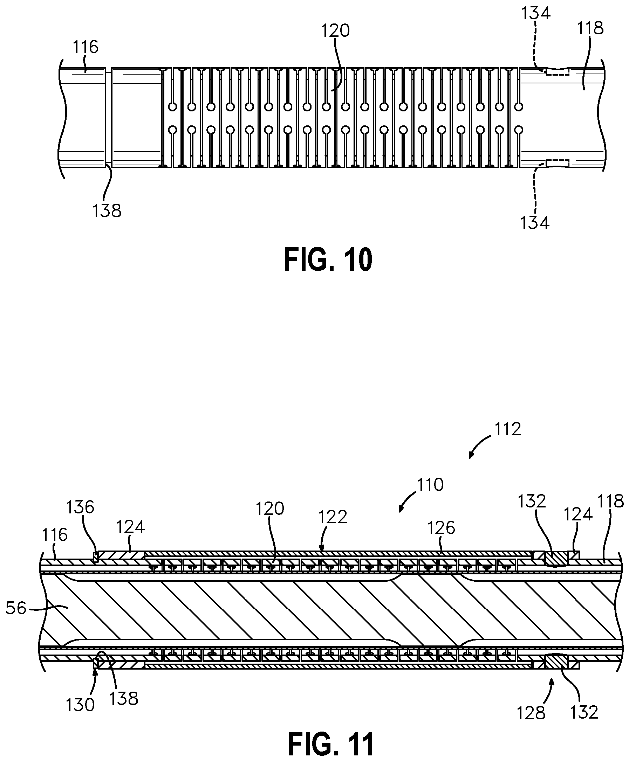

FIGS. 9 and 10 show one example of proximal actuator portion (116), spring (120), and distal actuator portion (118) in greater detail as singularly and unitarily formed together and incorporated generally into the inner tube of shaft assembly (14). To this end, spring (120) is formed by laser-cutting a pattern to remove material from clamp actuator (12) and increase the resilient bias between proximal actuator portion (116) and distal actuator portion (118) to form the resilient portion. It will be appreciated that any desired pattern may be so used for removal of material such that the invention is not intended to be unnecessarily limited to the pattern of removed material forming spring (120). Furthermore, while the present example shows proximal actuator portion (116), spring (120), and distal actuator portion (118) as singularly and unitarily formed together, an alternative resilient portion may be affixed between proximal actuator portion (116) and distal actuator portion (118) and thus assembled together rather than be singularly and unitarily formed together. The invention is thus not intended to be unnecessarily limited to the laser-cut spring (120) shown and described herein.

In use, with respect to FIG. 11, spacer (122) generally retains spring (120) preloaded in tension and, in turn, pins (132) urge spacer (122) in the proximal direction to seat spacer (122) against retaining ring (136) in compression such that spacer (122) is in a seated position. Thus, from the seated position, proximal and distal actuator portions (116, 118) are effectively coupled together and move simultaneously over the same distances during use. In other words, proximal and distal actuator portions (116, 118) move in a 1 to 1 ratio with spacer (122) in the seated position and the force transmitted through spring (120) being below the predetermined force. The position of clamp actuator (112) relative to waveguide (56) and the seated position of spacer (122) shown in FIG. 11 thus represents, in the present example, clamp arm (44) in the open position and free of any compressive clamping force between clamp arm (44) and blade (46) as shown in FIG. 3A.

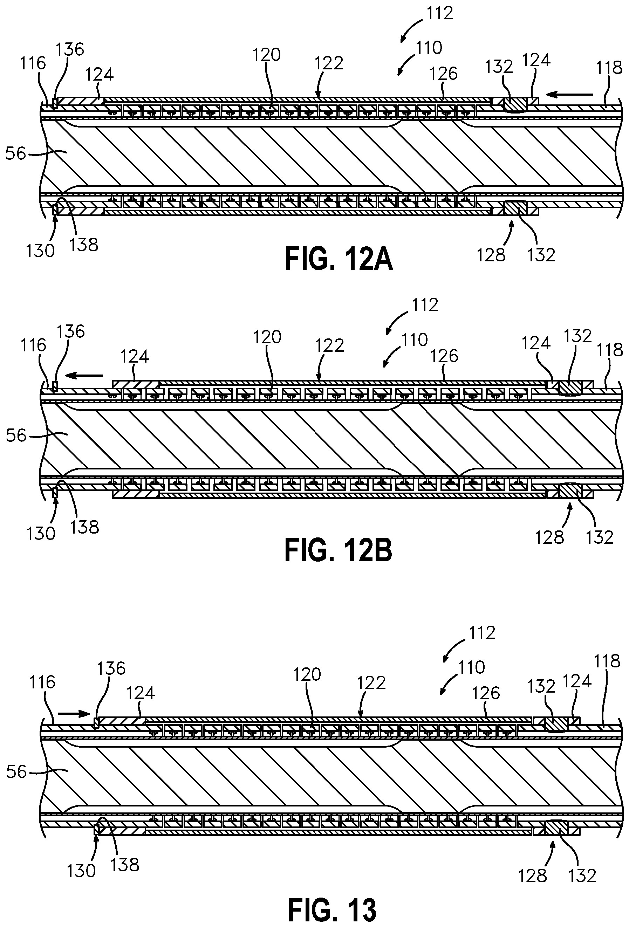

In conjunction with FIGS. 3A and 3B, FIGS. 12A and 12B show a closure stroke of clamp actuator (112) as clamp actuator (112) is pulled proximally to simultaneously direct clamp arm (44) from the open position toward the closed position. With spacer (122) seated against retaining ring (36) as shown in FIG. 12A, proximal actuator potion (116) draws on distal actuator portion (118) through spring (120) both simultaneously and over the same distances so long as force through the spring (120) remains below the predetermined force. As clamp arm (44) begins to compress against tissue while closing, the force being transferred through spring (120) may continue to increase. In the event that the applied force exceeds the predetermined force, the applied force effectively overcomes the preload in spring (120) and unseats spacer (122) such that proximal actuator portion (116) pulls away from distal actuator portion (118), no longer moving together with a 1 to 1 ratio. In other words, proximal and distal actuator portions (116, 118) are effectively decoupled from each other to generate relative movement between proximal and distal actuator portions (116, 118). For example, in the event that clamp arm (44) moving toward the closed position encounters a hard stop, both clamp arm (44) and distal actuator portion (118) will halt movement despite proximal actuator portion (116) continuing to be pulled proximally as shown in FIG. 12B. The relative movement between proximal and distal actuator portions (116, 118) upon deflection of spring (120) thereby limits the compressive force applied between clamp arm (44) and blade (46).

As discussed briefly above, with respect to FIGS. 3A and 3B, FIG. 13 shows clamp actuator (112) being pushed distally in order to open clamp arm (44) from the closed position toward the open position. Given such distal movement of proximal actuator portion (116), if not already seated, spacer (122) will seat against retaining ring (136). Once seated, continued distal movement of clamp actuator (112) moves retaining ring (136) to urge spacer (122) in the distal direction with pins (132) and distal actuator portion (118) to open clamp arm (44). Notably, in the distal direction, force transferred from proximal actuator portion (116) bypasses spring (120) and travels through spacer (122) to distal actuator portion (118). Spring (120) is thus not bearing any more or less loading than the set preload from spacer (122) while moving clamp arm toward the open position. Distal movement of proximal and distal actuator portions (116, 118) is thus always coupled through spacer in order to apply forces larger than the predetermined force so that the same end effector (16) may be used to pry apart various tissues by opening clamp arm (44).

While the present example of end effector (16) shows clamp arm (44) and blade (46) as respective jaws for compressing tissue while closing clamp arm (44) relative to blade (46) and prying apart tissues while opening clamp arm (44) relative to blade (46), it will be appreciated that such force limiter (110) may be used in other surgical instruments that incorporate alternative jaw arrangements with or without ultrasonic features. The invention is thus not intended to be unnecessarily limited to ultrasonic surgical instruments, whether robotic or hand held, as described herein.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A surgical instrument, comprising: (a) an end effector, comprising: (i) a jaw, and (ii) a clamp arm configured to move relative to the jaw between an open position and a closed position; and (b) a shaft assembly proximally and longitudinally extending from the end effector and including: (i) a clamp actuator operatively connected to the clamp arm and configured to selectively move longitudinally to selectively direct the clamp arm between the open and closed positions, wherein the clamp actuator includes: (A) a proximal actuator portion, (B) a distal actuator portion, and (C) a force limiter longitudinally connected between the proximal and distal actuator portions and configured to transfer movement from the proximal actuator portion to the distal actuator portion up to transferring a predetermined force therethrough, wherein at least a portion of the force limiter is configured to deflect upon exceeding the predetermined force such that the proximal actuator portion is configured to longitudinally move relative to the distal actuator portion and thereby limit a clamping force between the jaw and the clamp arm.

### Example 2

The surgical instrument of Example 1, wherein the force limiter is configured to secure the proximal actuator portion relative to the distal actuator portion while transferring movement from the proximal actuator portion to the distal actuator portion up to transferring the predetermined force therethrough.

### Example 3

The surgical instrument of any one or more of Examples 1 through 2, wherein the force limiter includes a resilient portion, and wherein the resilient portion, the proximal actuator portion, and the distal actuator portion are singularly and unitarily formed together.

### Example 4

The surgical instrument of Example 3, wherein the resilient portion is set in tension.

### Example 5

The surgical instrument of any one or more of Examples 1 through 4, wherein the clamp actuator is configured to selectively move a first longitudinal direction to selectively direct the clamp arm toward the closed position, wherein the clamp actuator is configured to selectively move a second longitudinal direction to selectively direct the clamp arm toward the open position, wherein the second longitudinal direction is opposite the first longitudinal direction.

### Example 6

The surgical instrument of Example 5, wherein the force limiter is configured to transfer movement in the first longitudinal direction from the proximal actuator portion to the distal actuator portion up to transferring the predetermined force therethrough such that the at least the portion of the force limiter deflects upon exceeding the predetermined force.

### Example 7

The surgical instrument of any one or more of Examples 5 through 6, wherein the force limiter is configured to transfer movement in the second longitudinal direction from the proximal actuator portion to the distal actuator portion such that the at least the portion of the force limiter does not deflect upon exceeding the predetermined force.

### Example 8

The surgical instrument of any one or more of Examples 5 through 7, wherein the first longitudinal direction is a proximal direction, and wherein the second longitudinal direction is a distal direction.

### Example 9

The surgical instrument of any one or more of Examples 5 through 8, wherein the force limiter further includes a resilient portion and a spacer, wherein the spacer is secured relative to the resilient portion to retain the resilient portion in tension.

### Example 10

The surgical instrument of any one or more of Example 9, wherein the force limiter further includes a first securement and a second securement, wherein the resilient portion has first and second opposing end portions, wherein the first securement is affixed to the first end portion of the resilient portion and configured to inhibit movement of the first end portion of the resilient portion relative to the spacer toward one of the first or second longitudinal directions, and wherein the second securement is affixed to the second end portion of the resilient portion and configured to inhibit movement of the second end portion of the resilient portion relative to the spacer toward each of the first and second longitudinal directions.

### Example 11

The surgical instrument of Example 10, wherein the spacer is compressed between the first and second securements while the force transferred through the resilient portion is less than the predetermined force such that that the spacer is against the first securement.

### Example 12

The surgical instrument of any one or more of Examples 10 through 11, wherein the spacer is offset from the first securement and remains affixed to the second securement while the force transferred through the resilient portion is greater than the predetermined force to accommodate deflection of the resilient portion.

### Example 13

The surgical instrument of any one or more of Examples 10 through 12, wherein the spacer is adjustable relative to the resilient portion to select a predetermined load tension in the resilient portion for adjusting the limit on the clamping force between the jaw and the clamp arm.

### Example 14

The surgical instrument of any one or more of Examples 1 through 13, wherein the jaw includes an ultrasonic blade and the shaft assembly includes an acoustic waveguide, and wherein the acoustic waveguide is operatively connected to the ultrasonic blade.

### Example 15

The surgical instrument of any one or more of Examples 1 through 14, further comprising a body, wherein the shaft assembly distally extends from the body to the end effector, and wherein the force limiter is positioned between the body and the end effector.

### Example 16

The surgical instrument of any one or more of Examples 1 through 15, wherein the shaft assembly further includes an outer sheath and an inner tube, and wherein the clamp actuator is incorporated into the inner tube.

### Example 17

A surgical instrument, comprising: (a) an end effector, comprising: (i) an ultrasonic blade, and (ii) a clamp arm configured to move relative to the ultrasonic blade between an open position and a closed position; and (b) a shaft assembly proximally and longitudinally extending from the end effector and including: (i) a clamp actuator operatively connected to the clamp arm and configured to selectively move longitudinally to selectively direct the clamp arm between the open and closed positions, wherein the clamp actuator includes: (A) a proximal actuator portion, (B) a distal actuator portion, and (C) a force limiter having a resilient portion longitudinally connected between the proximal and distal actuator portions and configured to transfer movement from the proximal actuator portion to the distal actuator portion up to transferring a predetermined force therethrough, wherein the resilient portion is configured to deflect upon exceeding the predetermined force such that the proximal actuator portion is configured to longitudinally move relative to the distal actuator portion and thereby limit a clamping force between the ultrasonic blade and the clamp arm, wherein the resilient portion, the proximal actuator portion, and the distal actuator portion are singularly and unitarily formed together.

### Example 18

The surgical instrument of Example 17, wherein the force limiter is configured to secure the proximal actuator portion relative to the distal actuator portion while transferring movement from the proximal actuator portion to the distal actuator portion up to transferring the predetermined force therethrough.

### Example 19

The surgical instrument of any one or more of Examples 17 through 18, wherein the clamp actuator is configured to selectively move a first longitudinal direction to selectively direct the clamp arm toward the closed position, wherein the clamp actuator is configured to selectively move a second longitudinal direction to selectively direct the clamp arm toward the open position, wherein the second longitudinal direction is opposite the first longitudinal direction.

### Example 20

The surgical instrument of any one or more of Examples 17 through 19, further comprising a body, wherein the shaft assembly distally extends from the body to the end effector, and wherein the force limiter is positioned between the body and the end effector.

### Example 21

A surgical instrument, comprising: (a) an end effector, comprising: (i) a jaw, and (ii) a clamp arm configured to move relative to the jaw between an open position and a closed position; and (b) a shaft assembly proximally and longitudinally extending from the end effector and including: (i) a clamp actuator operatively connected to the clamp arm and configured to selectively move longitudinally to selectively direct the clamp arm between the open and closed positions, wherein the clamp actuator includes: (A) a proximal actuator portion, (B) a distal actuator portion, and (C) a force limiter longitudinally connected between the proximal and distal actuator portions and configured to transfer movement from the proximal actuator portion to the distal actuator portion up to transferring a predetermined force therethrough, wherein the force limiter includes a resilient portion and a spacer, wherein the spacer is secured relative to the resilient portion to retain the resilient portion in tension.

### Example 22

A method of limit a clamping force between a jaw and a clamp arm of a surgical instrument, the surgical instrument including (a) an end effector, having: (i) a jaw, and (ii) a clamp arm configured to move relative to the jaw between an open position and a closed position; and (b) a shaft assembly proximally and longitudinally extending from the end effector and including (i) a clamp actuator operatively connected to the clamp arm and configured to selectively move longitudinally to selectively direct the clamp arm between the open and closed positions, wherein the clamp actuator includes: (A) a proximal actuator portion, (B) a distal actuator portion, and (C) a force limiter longitudinally connected between the proximal and distal actuator portions and configured to transfer movement from the proximal actuator portion to the distal actuator portion up to transferring a predetermined force therethrough, wherein at least a portion of the force limiter is configured to deflect upon exceeding the predetermined force such that the proximal actuator portion is configured to longitudinally move relative to the distal actuator portion and thereby limit a clamping force between the jaw and the clamp arm, the method comprising: (a) closing the clamp arm against a tissue between the clamp arm and the tissue with a clamping force; and (b) deflecting at least a portion of the force limiter thereby limiting the clamping force to no greater than a predetermined maximum clamping force.

### V. Miscellaneous

Any one or more of the teaching, expressions, embodiments, examples, etc. described herein may be combined with any one or more of the teachings, expressions, embodiments, examples, etc. described in U.S. Pat. App. No. 17/128,275, entitled "Ultrasonic Surgical Instrument with a Clamp Arm Clocking Assembly," filed on December 21, 2020; U.S. Pat. App. No. 16/556,661, entitled "Ultrasonic Surgical Instrument with a Multi-Planar Articulating Shaft Assembly," filed on August 30, 2019; U.S. Pat. App. No. 16/556,667, entitled "Ultrasonic Transducer Alignment of an Articulating Ultrasonic Surgical Instrument," filed on August 30, 2019; U.S. Pat. App. No. 16/556,625, entitled "Ultrasonic Surgical Instrument with Axisymmetric Clamping," filed on August 30, 2019; U.S. Pat. App. No. 16/556,635, entitled "Ultrasonic Blade and Clamp Arm Alignment Features," filed on August 30, 2019; U.S. Pat. App. No. 16/556,727, entitled "Rotatable Linear Actuation Mechanism," filed on August 30, 2019; U.S. Pat. App. No. 17/077,067, entitled "Surgical Instrument and Carrier KART Supporting Ultrasonic Transducer," filed on October 22, 2020; U.S. Pat. App. No. 17/077,086, entitled "Carrier KART and Jaw Closure of an Ultrasonic Surgical Instrument," filed on October 22, 2020; U.S. Pat. App. No. 17/077,130, entitled "Surgical Instrument with Clamping Sensor Feedback and Related Methods," filed on October 22, 2020; U.S. Pat. App. No. 17/077,136, entitled "Surgical Instrument with Non-clamping Sensor Feedback and Related Methods," filed on October 22, 2020; U.S. Pat. App. No. 17/077,250, entitled "Ultrasonic Surgical Instrument with a Carrier KART and Reusable Stage," filed on October 22, 2020; U.S. Pat. App. No. 17/077,373, entitled "Surgical Instrument with a Carrier KART and Various Communication Cable Arrangements," filed on October 22, 2020; U.S. Pat. App. No. 17/077,139, entitled "Ultrasonic Surgical Instrument with a Fixed Transducer Grounding," filed on October 22, 2020; U.S. Pat. App. No. 17/077,146, entitled "Ultrasonic Surgical Instrument with a Shaft Assembly and Elongated Waveguide Support Arrangement," filed on October 22, 2020; U.S. Pat. App. No. 17/077,152, entitled "Damping Rings for an Ultrasonic Surgical Instrument," filed on October 22, 2020; U.S. Pat. App. No. 17/077,110, entitled "Ultrasonic Surgical Instrument with a Mid-Shaft Closure System and Related Methods," filed on October 22, 2020; U.S. Pat. App. No. 17/076,956, entitled "Surgical Instrument with an Articulatable Shaft Assembly and Dual End Effector Roll," filed on October 22, 2020; U.S. Pat. App. No. 17/076,959, entitled "Ultrasonic Surgical Instrument with a Distally Grounded Acoustic Waveguide," filed on October 22, 2020; and/or U.S. Pat. App. No. 17/077,098, entitled "Ultrasonic Surgical Instrument with a Multiplanar Articulation Joint," filed on October 22, 2020. The disclosure of each of these applications is hereby incorporated by reference herein.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.
The following is a list of embodiments that may or may not be claimed hereinafter:
1. A surgical instrument, comprising:
   (a) an end effector, comprising:
      (i) a jaw, and
      (ii) a clamp arm configured to move relative to the jaw between an open position and a closed position; and
   (b) a shaft assembly proximally and longitudinally extending from the end effector and including:
      (i) a clamp actuator operatively connected to the clamp arm and configured to selectively move longitudinally to selectively direct the clamp arm between the open and closed positions, wherein the clamp actuator includes:
         (A) a proximal actuator portion,
         (B) a distal actuator portion, and
         (C) a force limiter longitudinally connected between the proximal and distal actuator portions and configured to transfer movement from the proximal actuator portion to the distal actuator portion up to transferring a predetermined force therethrough, wherein at least a portion of the force limiter is configured to deflect upon exceeding the predetermined force such that the proximal actuator portion is configured to longitudinally move relative to the distal actuator portion and thereby limit a clamping force between the jaw and the clamp arm.
2. The surgical instrument of Embodiment 1, wherein the force limiter is configured to secure the proximal actuator portion relative to the distal actuator portion while transferring movement from the proximal actuator portion to the distal actuator portion up to transferring the predetermined force therethrough.
3. The surgical instrument of Embodiment 1 or Embodiment 2, wherein the force limiter includes a resilient portion, and wherein the resilient portion, the proximal actuator portion, and the distal actuator portion are singularly and unitarily formed together.
4. The surgical instrument of Embodiment 3, wherein the resilient portion is set in tension.
5. The surgical instrument of any preceding Embodiment, wherein the clamp actuator is configured to selectively move a first longitudinal direction to selectively direct the clamp arm toward the closed position, wherein the clamp actuator is configured to selectively move a second longitudinal direction to selectively direct the clamp arm toward the open position, wherein the second longitudinal direction is opposite the first longitudinal direction.
6. The surgical instrument of Embodiment 5, wherein the force limiter is configured to transfer movement in the first longitudinal direction from the proximal actuator portion to the distal actuator portion up to transferring the predetermined force therethrough such that the at least the portion of the force limiter deflects upon exceeding the predetermined force.
7. The surgical instrument of Embodiment 5 or Embodiment 6, wherein the force limiter is configured to transfer movement in the second longitudinal direction from the proximal actuator portion to the distal actuator portion such that the at least the portion of the force limiter does not deflect upon exceeding the predetermined force.
8. The surgical instrument of any one of Embodiments 5 - 7, wherein the first longitudinal direction is a proximal direction, and wherein the second longitudinal direction is a distal direction.
9. The surgical instrument of any one of Embodiments 5 - 8, wherein the force limiter further includes a resilient portion and a spacer, wherein the spacer is secured relative to the resilient portion to retain the resilient portion in tension.
10. The surgical instrument of Embodiment 9, wherein the force limiter further includes a first securement and a second securement, wherein the resilient portion has first and second opposing end portions, wherein the first securement is affixed to the first end portion of the resilient portion and configured to inhibit movement of the first end portion of the resilient portion relative to the spacer toward one of the first or second longitudinal directions, and wherein the second securement is affixed to the second end portion of the resilient portion and configured to inhibit movement of the second end portion of the resilient portion relative to the spacer toward each of the first and second longitudinal directions.
11. The surgical instrument of Embodiment 10, wherein the spacer is compressed between the first and second securements while the force transferred through the resilient portion is less than the predetermined force such that that the spacer is against the first securement.
12. The surgical instrument of Embodiment 10 or Embodiment 11, wherein the spacer is offset from the first securement and remains affixed to the second securement while the force transferred through the resilient portion is greater than the predetermined force to accommodate deflection of the resilient portion.
13. The surgical instrument of any one of Embodiments 9 - 12, wherein the spacer is adjustable relative to the resilient portion to select a predetermined load tension in the resilient portion for adjusting the limit on the clamping force between the jaw and the clamp arm.
14. The surgical instrument of any preceding Embodiment, wherein the jaw includes an ultrasonic blade and the shaft assembly includes an acoustic waveguide, and wherein the acoustic waveguide is operatively connected to the ultrasonic blade.
15. The surgical instrument of any preceding Embodiment, further comprising a body, wherein the shaft assembly distally extends from the body to the end effector, and wherein the force limiter is positioned between the body and the end effector.
16. The surgical instrument of any preceding Embodiment, wherein the shaft assembly further includes an outer sheath and an inner tube, and wherein the clamp actuator is incorporated into the inner tube.
17. A surgical instrument, comprising:
   (a) an end effector, comprising:
      (i) an ultrasonic blade, and
      (ii) a clamp arm configured to move relative to the ultrasonic blade between an open position and a closed position; and
   (b) a shaft assembly proximally and longitudinally extending from the end effector and including:
      (i) a clamp actuator operatively connected to the clamp arm and configured to selectively move longitudinally to selectively direct the clamp arm between the open and closed positions, wherein the clamp actuator includes:
         (A) a proximal actuator portion,
         (B) a distal actuator portion, and
         (C) a force limiter having a resilient portion longitudinally connected between the proximal and distal actuator portions and configured to transfer movement from the proximal actuator portion to the distal actuator portion up to transferring a predetermined force therethrough, wherein the resilient portion is configured to deflect upon exceeding the predetermined force such that the proximal actuator portion is configured to longitudinally move relative to the distal actuator portion and thereby limit a clamping force between the ultrasonic blade and the clamp arm,
         wherein the resilient portion, the proximal actuator portion, and the distal actuator portion are singularly and unitarily formed together.
18. The surgical instrument of Embodiment 17, wherein the force limiter is configured to secure the proximal actuator portion relative to the distal actuator portion while transferring movement from the proximal actuator portion to the distal actuator portion up to transferring the predetermined force therethrough.
19. The surgical instrument of Embodiment 17 or Embodiment 18, wherein the clamp actuator is configured to selectively move a first longitudinal direction to selectively direct the clamp arm toward the closed position, wherein the clamp actuator is configured to selectively move a second longitudinal direction to selectively direct the clamp arm toward the open position, wherein the second longitudinal direction is opposite the first longitudinal direction.
20. The surgical instrument of any one of Embodiments 17 - 19, further comprising a body, wherein the shaft assembly distally extends from the body to the end effector, and wherein the force limiter is positioned between the body and the end effector.
21. A surgical instrument, comprising:
   (a) an end effector, comprising:
      (i) a jaw, and
      (ii) a clamp arm configured to move relative to the jaw between an open position and a closed position; and
   (b) a shaft assembly proximally and longitudinally extending from the end effector and including:
      (i) a clamp actuator operatively connected to the clamp arm and configured to selectively move longitudinally to selectively direct the clamp arm between the open and closed positions, wherein the clamp actuator includes:
         (A) a proximal actuator portion,
         (B) a distal actuator portion, and
         (C) a force limiter longitudinally connected between the proximal and distal actuator portions and configured to transfer movement from the proximal actuator portion to the distal actuator portion up to transferring a predetermined force therethrough, wherein the force limiter includes a resilient portion and a spacer, wherein the spacer is secured relative to the resilient portion to retain the resilient portion in tension.

## Claims

1. A surgical instrument, comprising:
(a) an end effector, comprising:
(i) a jaw, and
(ii) a clamp arm configured to move relative to the jaw between an open position and a closed position; and
(b) a shaft assembly proximally and longitudinally extending from the end effector and including:
(i) a clamp actuator operatively connected to the clamp arm and configured to selectively move longitudinally to selectively direct the clamp arm between the open and closed positions, wherein the clamp actuator includes:
(A) a proximal actuator portion,
(B) a distal actuator portion, and
(C) a force limiter longitudinally connected between the proximal and distal actuator portions and configured to transfer movement from the proximal actuator portion to the distal actuator portion up to transferring a predetermined force therethrough, wherein at least a portion of the force limiter is configured to deflect upon exceeding the predetermined force such that the proximal actuator portion is configured to longitudinally move relative to the distal actuator portion and thereby limit a clamping force between the jaw and the clamp arm.

2. The surgical instrument of claim 1, wherein the force limiter is configured to secure the proximal actuator portion relative to the distal actuator portion while transferring movement from the proximal actuator portion to the distal actuator portion up to transferring the predetermined force therethrough.

3. The surgical instrument of claim 1 or claim 2, wherein the force limiter includes a resilient portion, and wherein the resilient portion, the proximal actuator portion, and the distal actuator portion are singularly and unitarily formed together, optionally wherein the resilient portion is set in tension.

4. The surgical instrument of any preceding claim, wherein the clamp actuator is configured to selectively move a first longitudinal direction to selectively direct the clamp arm toward the closed position, wherein the clamp actuator is configured to selectively move a second longitudinal direction to selectively direct the clamp arm toward the open position, wherein the second longitudinal direction is opposite the first longitudinal direction.

5. The surgical instrument of claim 4, wherein the force limiter is configured to transfer movement in the first longitudinal direction from the proximal actuator portion to the distal actuator portion up to transferring the predetermined force therethrough such that the at least the portion of the force limiter deflects upon exceeding the predetermined force.

6. The surgical instrument of claim 4 or claim 5, wherein the force limiter is configured to transfer movement in the second longitudinal direction from the proximal actuator portion to the distal actuator portion such that the at least the portion of the force limiter does not deflect upon exceeding the predetermined force.

7. The surgical instrument of any one of claims 4 - 6, wherein the first longitudinal direction is a proximal direction, and wherein the second longitudinal direction is a distal direction.

8. The surgical instrument of any one of claims 4 - 7, wherein the force limiter further includes a resilient portion and a spacer, wherein the spacer is secured relative to the resilient portion to retain the resilient portion in tension.

9. The surgical instrument of claim 8, wherein the force limiter further includes a first securement and a second securement, wherein the resilient portion has first and second opposing end portions, wherein the first securement is affixed to the first end portion of the resilient portion and configured to inhibit movement of the first end portion of the resilient portion relative to the spacer toward one of the first or second longitudinal directions, and wherein the second securement is affixed to the second end portion of the resilient portion and configured to inhibit movement of the second end portion of the resilient portion relative to the spacer toward each of the first and second longitudinal directions, optionally wherein the spacer is compressed between the first and second securements while the force transferred through the resilient portion is less than the predetermined force such that that the spacer is against the first securement.

10. The surgical instrument of claim 9, wherein the spacer is offset from the first securement and remains affixed to the second securement while the force transferred through the resilient portion is greater than the predetermined force to accommodate deflection of the resilient portion.

11. The surgical instrument of any one of claims 8 - 10, wherein the spacer is adjustable relative to the resilient portion to select a predetermined load tension in the resilient portion for adjusting the limit on the clamping force between the jaw and the clamp arm.

12. The surgical instrument of any preceding claim, wherein the jaw includes an ultrasonic blade and the shaft assembly includes an acoustic waveguide, and wherein the acoustic waveguide is operatively connected to the ultrasonic blade.

13. The surgical instrument of any preceding claim, further comprising a body, wherein the shaft assembly distally extends from the body to the end effector, and wherein the force limiter is positioned between the body and the end effector.

14. The surgical instrument of any preceding claim, wherein the shaft assembly further includes an outer sheath and an inner tube, and wherein the clamp actuator is incorporated into the inner tube.

15. A surgical instrument, comprising:
(a) an end effector, comprising:
(i) a jaw, and
(ii) a clamp arm configured to move relative to the jaw between an open position and a closed position; and
(b) a shaft assembly proximally and longitudinally extending from the end effector and including:
(i) a clamp actuator operatively connected to the clamp arm and configured to selectively move longitudinally to selectively direct the clamp arm between the open and closed positions, wherein the clamp actuator includes:
(A) a proximal actuator portion,
(B) a distal actuator portion, and
(C) a force limiter longitudinally connected between the proximal and distal actuator portions and configured to transfer movement from the proximal actuator portion to the distal actuator portion up to transferring a predetermined force therethrough, wherein the force limiter includes a resilient portion and a spacer, wherein the spacer is secured relative to the resilient portion to retain the resilient portion in tension.
